# EUROPEAN PATENT APPLICATION

(11) **EP 2 082 756 A1**
(43) Date of publication of application: **29.07.2009**
(21) Application number: 07425783.3
(22) Date of filing: 10.12.2007
(51) Int. Cl.: A61L 31/14, A61N 1/05, A61N 1/375

(54) **Sealing process for electronics surgical implants**

(30) Priority: 11.12.2006 IT RM20060669
(71) Applicant: Romana Film Sottili Srl, Carsoli (AQ) (IT)
(72) Inventor: Misiano, Carlo, 00161 Roma (IT)

(57) **Abstract**

The invention relates a sealing system to make completely impermeable, in respect to the corporal fluid, biocompatible and, if necessary, a faster osteointegration, an electronic surgical prosthesis, in particular an endococleare prosthesis. In particular the invention permits to reduce the prosthesis thickness with notable advantage, when it is inserted in the temporal bone in subjects frequently very young. It improves the actual solutions which are based on the sealed ceramic containers. An other advantage of the invention lies in the cost reduction of the prosthesis sealing caused by the high cost of the actual ceramic containers. In addition the invention presents the advantage to favour, when necessary a faster surgical prosthesis osteointegration. The invention lies in the field of the human necessities and in the field of the surgical prosthesis manufacture.

## Description

The invention relates a process to make totally impermeable, in respect of body fluids, biocompatible and , if necessary, of faster osteointegration, an electronic prosthesis, particularly an intracochlear. The invention is able to reduce the thickness of the prosthesis, offering a remarkable advantage, having to be inserted into the temporal bone of frequently very young subjects. The invention improves the actually employed solution that are based on the use of sealed ceramic container. A further advantage of the invention resides in the cost reduction of the prosthesis sealing, in fact, the cost of the current ceramic container is very high. In addition, the invention offers the advantage to permit, when necessary, a more quick osteointegration of the surgical prosthesis.

The invention lies in the field of the human necessities and in the field of the surgical prosthesis.

The invention is described in the following, to be illustrated and not limited, referring to the enclosed figures and relating to a preferred version by the inventor.

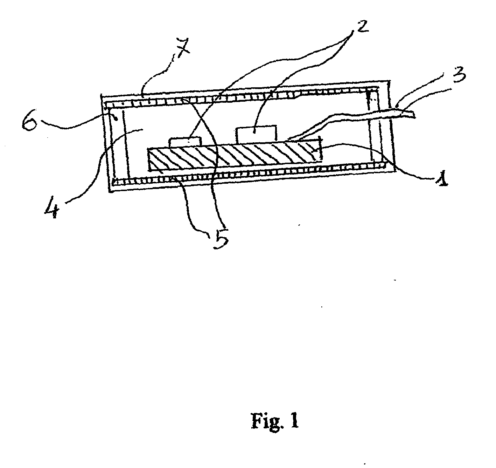

Fig. 1 - Schematic representation of the prosthesis sealed by the proposed process.

In the figure are visible:
1 Substrate of electronic circuit;
2 Discrete elements bonded on the electronic circuit;
3 Connections;
4 Resin;
5 Microsheets;
6 Frame;
7. Thin film for biocompatibility and osteointegration.

The Fig. 1 represents the sealing method where the substrate of the electronic circuit 1 can be constituted by a semiconductor or a ceramic substrate on which are situated integrated circuital elements and discrete elements 2. From this circuit the connections 3 come out. The resin 4, that represents one of the innovative aspects of the invention, has the aim of planarizing the surface of the circuit structure, to seal the exit of the connections and, finally, to bond the microsheets 5, that can be constituted by glass, quartz or other thin dielectric material, and the frame 6. On the microsheets 5 upper and lower, in respect to the microcircuit, and also on the frame 6, is deposited, even under vacuum, an thin film 7 of biocompatible material, as for example, TiO₂, Titanium dioxide, or, if necessary, a thin film also for osteointegration.

The frame can be constituted either by glass or by any material on condition that it can guarantee the impermeability and permit the connections exit to be electrically insulated. The same resin can be also utilized as lateral seal. Said resin 4 has been chosen for its low vapour pressure, when polymerised and for its hardness and in addition, also for its adhesivity to the two micro-sheets 5 and to the frame 6 to which it gives also the necessary rigidity.

The product, actually preferred by the inventor, is high vacuum resin, for instance TorrSeal, easily to find on the market. The thickness of the micro-sheets 5 is about one tenth of millimeter.

## Claims

1. Sealing system to make impermeable electronic surgical implants, **characterized by** the fact to be constituted by micro-sheets (5) assembled by a resin (4) which is also its planarizing structure, in respect of the discrete elements (2) assembled on the electronic circuit substrate (1), and the sealing connections (3), being the lateral part of the sealing structure composed by frame (6) and being the external surface covered by a biocompatible and osteous-integrating thin film (7).

2. Sealing process to make impermeable electronic surgical implants, as for Claim 1,, **characterized by** the fact that the frame (6) can be constituted also by the resin (4) only, that is, it can be constituted by a frame of adapt material to permit the going out of the connections (3) electrically insulated.

3. Sealing process to make impermeable electronic surgical implants, as for Claim 1, **characterized by** the fact that the surgical implant covered by a thin film (7) biocompatible and/or osteointegrating can be deposited under vacuum.

4. Sealing process to make impermeable electronic surgical, as for Claim 1, **characterized by** the fact that the micro-sheets (5) can be realized by glass, quarts, fused silica or other dielectric material adapt to insulate the electronic circuit in respect of the corporal fluids.

5. Sealing process to make impermeable electronic surgical, as for Claim 1, **characterized by** the fact that the surgical prosthesis can be also an intracoclear device.
